# EUROPEAN PATENT SPECIFICATION

(11) **EP 2 214 727 B1**
(45) Date of publication and mention of the grant of the patent: **11.04.2012**
(21) Application number: 07866765.6
(22) Date of filing: 20.11.2007
(51) Int. Cl.: A61L 9/14, B05B 17/06

(54) **NEBULISING DEVICE FOR LIQUID SUBSTANCES**
VERNEBLER FÜR FLÜSSIGE SUBSTANZEN
DISPOSITIF NÉBULISEUR POUR SUBSTANCES LIQUIDES

(43) Date of publication of application: 11.08.2010
(73) Proprietor: ZOBELE HOLDING S.P.A., 38100 Trento (IT)
(72) Inventor: MARCHETTI, Fabio, I-38050 Povo (IT); DEFLORIAN, Stefano, I-38040 Martignano (IT); FAES, Alessandro, I-38040 Martignano (IT); PAOLAZZI, Gianluca, I-38030 Giovo (IT)
(74) Representative: Faggioni, Carlo Maria
(86) International application number: PCT/IT2007/000813
(87) International publication number: WO 2009/066329

(56) References cited:
- EP-A- 0 897 755
- WO-A-03/068413
- WO-A-03/099458
- WO-A-2007/054920
- WO-A-2007/120489
- US-A1- 2006 011 739

## Description

### Technical Field

This invention concerns a nebulising element for liquid substances_{.}

### State of the Art

The devices currently found on sale to diffuse perfumed fragrances, insecticides, medicines or aromatherapy oils into the environment preferably use emanation systems that issue ultrasounds see eg: WO0/3068413 or US2006/0011739.

Using such devices it is possible to issue a set amount of a nebulised product into the environment without altering its chemical composition and at a limited cost in terms of power.

These devices generally include a reservoir containing the substance to be nebulised, a porous element or wick, which is partly immersed inside the reservoir, and a nebulising element, to which the porous element conveys the liquid to be diffused. The nebulising element includes a microperforated membrane, which is made to vibrate at a suitable oscillation frequency by an associated piezoelectric element. The piezoelectric element is in turn connected to an electronic circuit which, when powered from an outside source, such as a battery for example, causes said piezoelectric element to change in terms of dimensions.

The membrane and the piezoelectric element must be perfectly coupled, for example, using suitable connectors or by integrating the piezoelectric element directly into the membrane so that the movement caused by dimensional variations of the piezoelectric material is totally transferred to the membrane. The presence of the piezoelectric element must in no way interfere with the oscillation of the membrane.

The oscillation of the membrane nebulises the liquid substance into the environment, without altering it chemically.

The membrane can also be connected to one or more oscillation sensors, beside the container, which regulate membrane oscillation frequency.

The effectiveness of ultrasonic nebulisation is linked to different factors such as, for example, the physical and chemical properties of the liquid, the type and size of the holes in the membrane and the membrane's oscillation frequency. This latter characteristic, which has a notable influence, not only depends on the characteristics of the membrane (material, thickness, etc....), but also on the damping elements around it, such as for example, supporting elements or elements connecting to the other parts of the device.

Even interference of the porous element with the nebulising element will change the distribution of the membrane masses and/or cause a shift in the system's resonance, preventing it from vibrating at optimum frequency or in any case, at a constant frequency that is little influenced by external factors which can be difficult to control, such as the length of the porous element, for example. If the system does not operate at its resonance frequency, the oscillation amplitude of the membrane will vary and this can lead to non-optimum or ineffective nebulisation.

In fact, once the maximum dimensions for the holes in the membrane have been established and therefore, the size of the liquid particles to be diffused, the greater the oscillation of the membrane, the more mechanical energy will be transferred to the liquid particles projected into the environment and the further the liquid will be nebulised.

Some devices have partially overcome the problems arising from interference between the porous element and the membrane, by placing the porous section so it is offset compared to the membrane axis. However, this solution has failed to solve the problem completely, above all on account of the difficulties encountered in coupling the nebulising and porous elements.

The direct consequence of this ineffective coupling is a reduced flexibility in the nebulisation system and a lack of nebulising efficiency.

Therefore, the Applicant has found that the solutions used in known techniques can be improved from both a structural viewpoint and in terms of operation, overall dimensions, flexibility and effective nebulisation.

### Purpose of the Invention

The purpose of this invention is to supply an element to nebulise liquid substances, which is able to eliminate the problems described here above.

Specifically, the aim of this invention is to supply a nebulising element for liquid substances that is able to be coupled stably to the liquid procurement means, such as a porous element for example, without changing the vibration frequency of the whole nebulising system.

Moreover, it is the purpose of this invention to create a nebulising element for liquid substances that allows effective and constant liquid substance diffusion into the environment.

Lastly, the purpose of this invention is to create a nebulising element for liquid substances that is structurally simple, small in size and easily industrialised. It must also have limited costs and be easily adaptable to the various types of emanation devices.

According to this invention, a nebulising element for liquid substances is made with the characteristics present in one or more of the annexed claims.

### Brief Description of the Drawings

The invention will be described with reference to the annexed drawings, illustrating an embodiment example that is in no way limited, in which:
- Figure 1 shows an exploded view of an initial configuration of a nebulising element for liquid substances according to this invention;
- Figure 2 shows an exploded view of a second configuration of a nebulising element for liquid substances according to this invention;

- Figures 3-7 show different embodiment variations of the nebulising element illustrated in Figure 1;
- Figure 8 shows a rear perspective view of the nebulising element that is the subject of this invention;
- Figure 9a shows a first configuration of the nebulising element illustrated in Figure 3 coupled with a porous element;
- Figure 9b shows a second configuration of the nebulising element illustrated in Figure 4 coupled with a porous element;
- Figures 9c-9e show other coupling configurations between the nebulising element and the porous element;
- Figure 10 shows the nebulising element illustrated in Figure 2 that can be coupled with two different porous elements;
- Figure 11 shows a perspective view (exploded) of a first embodiment of a device to emanate liquid substances, including the nebulising element that is the subject of this invention;
- Figure 12 shows a perspective view (exploded) of a second embodiment of a device to dispense liquid substances, including the nebulising element that is the subject of this invention;
- Figures 13a and 13b each show a partially sectioned side view of another two alternative embodiments of the nebulising element that is the subject of this invention;
- Figure 14 shows another embodiment of the nebulising element, associated with an elastic element.

### Detailed Description of the Preferred Embodiments of the Invention

With reference to the annexed figures, 1 has been used to indicate, overall, a nebulising element for liquid substances that is the subject of this invention.

The nebulising element 1 includes at least a first 6 and a second 7 support, which can be placed over one another and defining a container 8 on their interior.

Inside the container 8, the nebulising element 1 includes at least one membrane 9 coupled to a piezoelectric element 10, which is in turn connected to an electronic circuit 20.

This latter, which can be electrically connected to a power source, such as a battery 22 for example, causes the piezoelectric element 10 to change size. These controlled deformations within the piezoelectric element 10 cause the membrane 9 to vibrate. The special coupling between the membrane 9 and the piezoelectric element 10 causes the membrane 9 to vibrate along an axis 9a which is perpendicular to the surface on which the membrane 9 rests.

The electronic circuit 20 needs to guarantee a suitable pulse to the piezoelectric material in terms of intensity, duration, form and frequency, to foster the correct excitement of the material forming the piezoelectric element 10.

The nebulising element 1 can be coupled to a liquid procurement means 5, such as for example, a porous element impregnated with a liquid to be diffused, contained inside a reservoir 4.

For the sake of simplicity, when continuing with the description, reference will be made to a porous element 5 instead of a liquid procurement means. The porous element 5 is preferably to be made in fibre, glass fibre, porous plastic or in a similar material with a porousness somewhere between 30% and 80%, preferrably 50%.

The fluid is channelled between the nebulising element 1 and the porous element 5; in other words, the liquid from the porous element 5 flows into the nebulising element 1, via capillary action, until it reaches the proximity of the membrane 9.

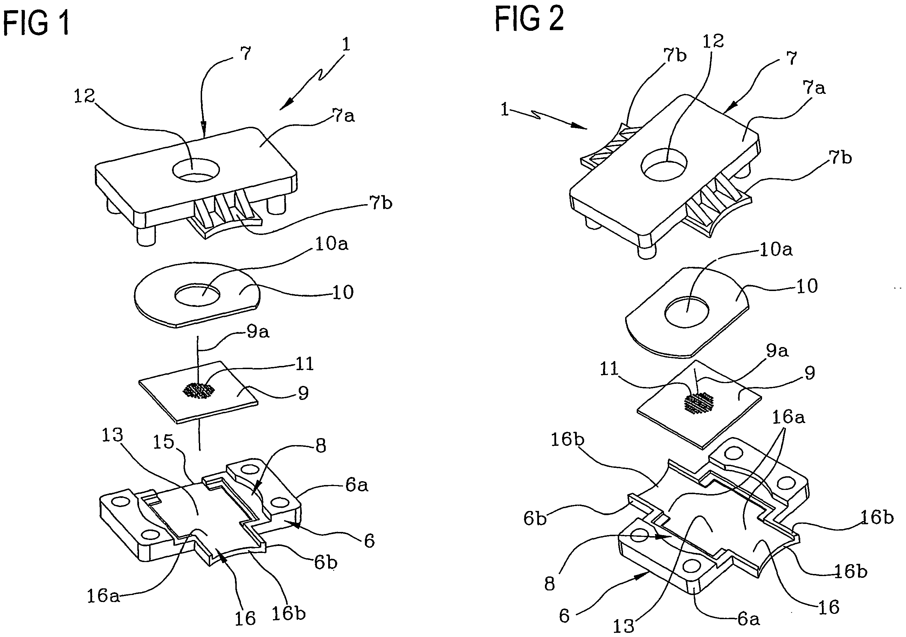

As can be clearly seen in Figures 1 and 2, the membrane 9 includes - preferably in a central position - at least one area 11 comprised of a number of small through holes.

These holes, which preferably have a round or square section, have a maximum cross dimensions such as a side or diameter for example, preferably between 5 and 20 mµ, with a density, in the case of holes with a 10 mµ diameter, for example, of 50 holes per square millimetre. The membrane 9 is preferably made in metal, plastic or even better, in silicon, quartz or glass and may be of extremely reduced thickness, between 10 and 100 mµ - preferably between 30 and 70 mµ, In the case of a membrane 9 in silicon, it is best to make it by covering the outer surfaces of the membrane with a layer of silicon nitrides or silicon oxides with passivation action, or with a layered combination of these substances, followed by lithography and subsequent chemical attack (bath in a basic solution for anisotropic etching) or physical attack (deep reactive ion etching, reactive ion etching) to make the holes in the membrane. Subsequent further chemical and physical attacks will make the fine substrate finer, until a membrane 9 of the required thickness is obtained.

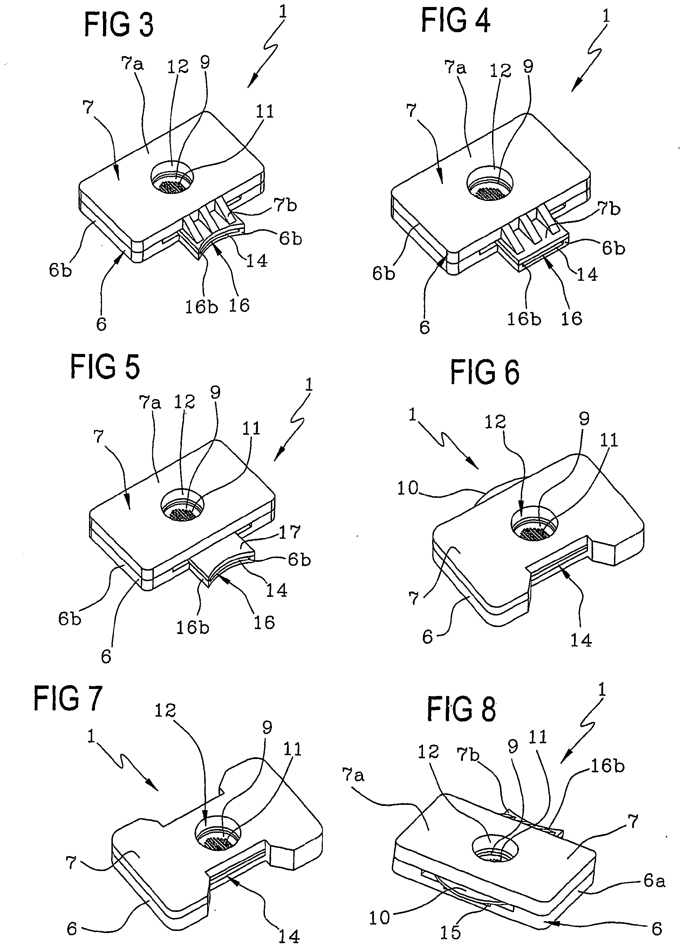

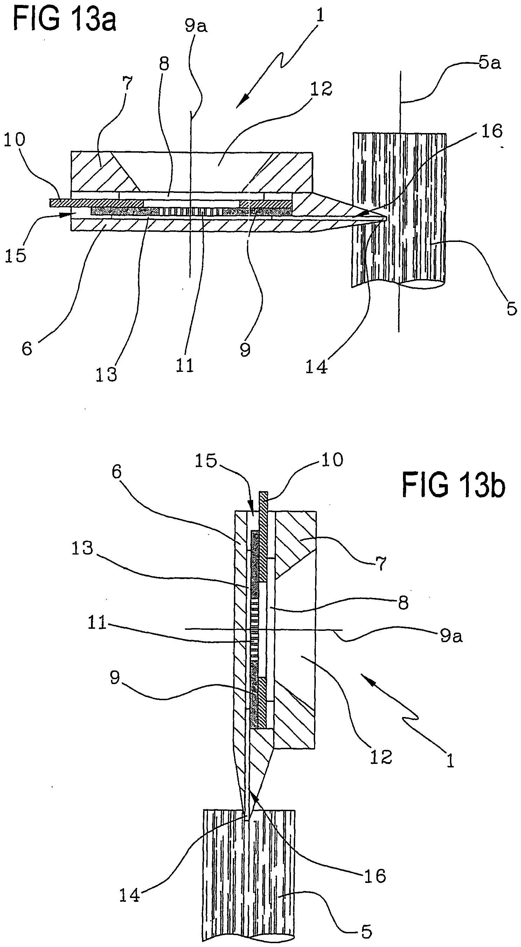

The second support 7 presents an opening 12, preferably in a central position, which, when the nebulising element 1 is fully assembled, is placed level with the perforated area 11 of the membrane 9, so as to emit the nebulised liquid. This opening 12 may have parallel sides as illustrated in Figures 3-8, or reciprocally slanted sides, preferably flared as illustrated in the Figures 13a and 13b, to aid the emission of particles and prevent the condensation from forming.

The nebulising element 1 is therefore made by placing the following onto one another in sequence: the first support 6; the membrane 9; the piezoelectric element 10 and the second support 7, as illustrated in the Figures 1 and 2.

To aid the assembly stage, the piezoelectric element 10 is suitably shaped, at least around the edges, as can be seen in Figures 1 and 2. It is preferable for the piezoelectric element 10 to have a ring shape with a hollow central section 10a, coupled by placing it over the perforated central area 11 of the membrane 9.

The piezoelectric element 10 is connected to the membrane 9 by special connecting elements; for example, the piezoelectric element 10 can be glued, soldered or directly integrated to the membrane 9. The electronic circuit 20 is connected to the piezoelectric element 10 by electrical connections 23 which, for example, are directly soldered to the piezoelectric element 10 or connected electrically to it via mechanical interference obtained with the supports 6 and 7 respectively. Lastly, the membrane 9 and piezoelectric element 10 are enclosed between the two supports 6 and 7. Between the first support 6 and the membrane 9 there is a collector 13 to collect the liquid which, on account of capillary action flows from the porous element 5 to the collector 13 itself. More precisely, the collector 13 is created on the first support 6, and in particular, on its upper surface, which is delimited at the top by the membrane 9. The collector 13 is an area of limited size, created beneath the membrane 9, between the membrane 9 itself and the first support 6. It has a height of between 5 and 150 mµ (preferably, between 20 and 100 mµ) and lateral dimensions that can vary between 3 and 6 mm (preferably between 4 and 5 mm). What counts is that, in any case, the lateral dimensions of the collector 13 are less than or at most, equal to the lateral dimensions of the membrane 9.

Therefore, the dimensions of the liquid collector 13 are such that they allow the procurement of liquid inside the collector 13 exclusively by capillary action.

The nebulising element 1 includes at least one capillary opening 14, which can be seen in the Figures 3-7, to allow the liquid for nebulising to enter the collector 13; it also has at least one outlet opening 15, which can be seen in Figures 1, 2 and 8, to allow air circulation and discharge and to facilitate fluid movement through capillary action.

In an initial embodiment, illustrated in Figures 6 and 7, both the capillary opening 14 for incoming liquid and the air outlet opening 15 are situated so that they are at least level with a perimeter portion of the collector 13. This means that each one will preferably delimit at least one side of the collector 13. In these configurations, the porous element 5 takes up position against the capillary opening 14 that makes it easier for the liquid on the surface of the porous element 5 to enter directly into the collector 13.

Advantageously, the nebulising element 1, in the preferred configuration, as illustrated in the formats shown in Figures 3, 4, 5 and 8, includes a capillary channel 16 that branches from the collector 13 to place it in fluid communication with the porous element 5.

The capillary channel 16 includes a first end 16a communicating with the liquid collector 13, and a second end 16b, opposite to the first, which can be coupled to the porous element 5. In this configuration, the capillary opening 14 for liquid coming into the collector 13 of the nebulising element 1, is located level with the second end 16b of the capillary channel 16.

In this case too, the discharge opening 15 can be positioned level with a perimeter side of the collector 13, as shown in Figure 8. The capillary opening for incoming liquid 14 and the air discharge outlet 15 can be located along the same axis - as shown in the annexed figures - or out of line with one another. It is preferable for the two openings 14 and 15 to remain coplanar, whether they are located along the same axis or out of line.

In an initial layout, the capillary channel 16, illustrated in Figures 1 and 2, is at least partially defined by the second support 7 and the first 6 support; specifically, in Figures 1 and 2 it can clearly be seen that the channel 16 is created in the top surface of the first support 6.

In particular, with reference to Figures 3, 4 and 8, the first 6 and second 7 supports are each comprised of a main body 6a, 7a, which is polygonal or circular in shape, and at least one respective appendage 6b, 7b projecting from its main body 6a, 7a, and with which it is coplanar.

The capillary channel 16 is defined by the coupled appendages 6b, 7b of the first 6 and second 7 support.

Alternatively, as shown in Figure 5, only the first support 6 has an appendage 6a and therefore, in this case the capillary channel 16 is at least partially defined by the first support 6 and by at least one covering plate 17, preferably in metal.

To allow the liquid to travel through the capillary channel 16 and therefore, inside the collector 13, the capillary channel 16 and in particular, its second end 16b, is in extremely close contact with the porous element 5. In other words, the second end 16b of the capillary channel 16 is at least partially resting against a coupling surface of the porous element 5.

Depending to the shape of the coupling surfaces on the porous element 5, the second end 16b of the capillary channel 16, or the capillary opening 14 for incoming liquid is shaped to adapt to said form.

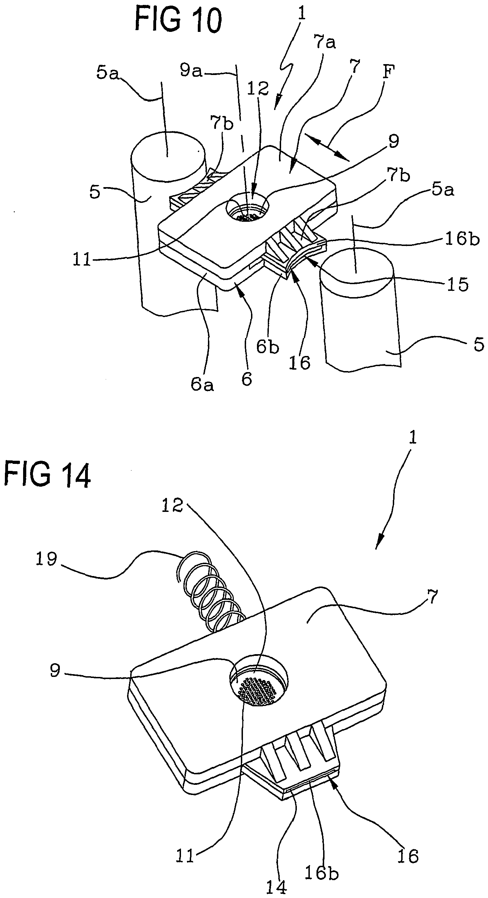

As shown in the annexed Figures, the capillary channel may be in different shapes, for example, trapezoidal (Figure 14) or rectangular (Figure 4). The second end 16b of the capillary channel 16 may be formed differently: for example, it may be arched (Figures 3 and 5), to follow the side surface of the cylindrical porous element 5 better; or straight (Figures 4 and 14), for use with porous elements with coupling surfaces that are curved or straight. To make sure that liquid flows into the collector 13 by capillary action, at least one part of the capillary opening 14 must be in intimate contact with the surface of the porous element 5.

In the case where there is no capillary channel 16 (Figures 6 and 7) too, the capillary opening 14 may have a straight or curved profile.

At least one part of the second end 16b of the capillary channel 16, and therefore, at least one part of the capillary opening 14, needs to rest upon and exert a slight pressure against the porous element 5, to facilitate liquid input into the channel 16 by means of capillary action.

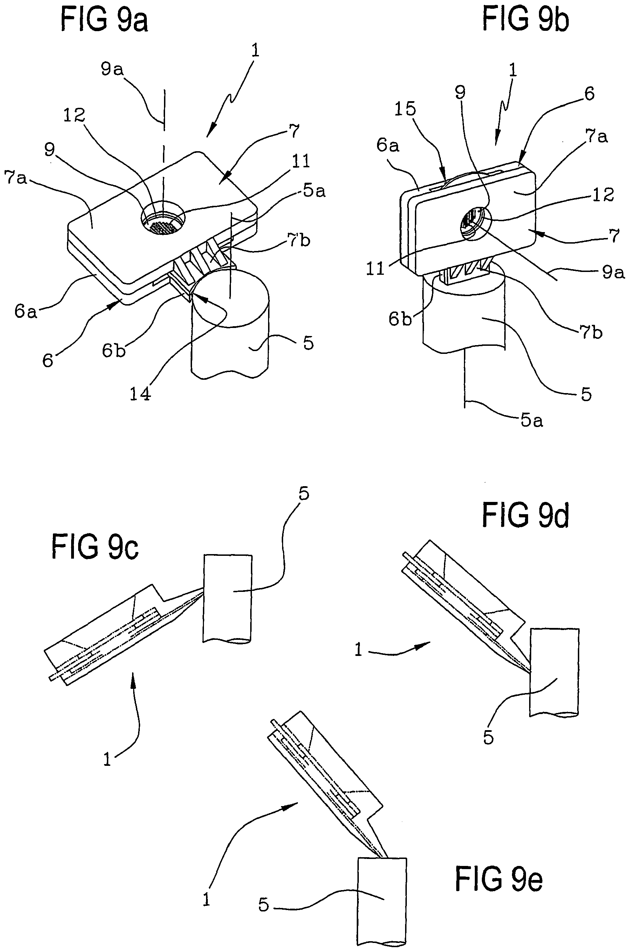

To this end, the nebulising element 1 is beneficially coupled to elastic bodies 19 - schematically illustrated in Figure 14 - comprised, for example, of elastic frames or pressure springs. These elastic bodies 19 will keep the nebulising element 1 in position against the porous element 5, guaranteeing constant contact between the capillary opening 14 and the coupling surface of the porous element 5 over time. The relevant position of the nebulising element 1 compared to the porous element 5 can be changed as required. Figures 9a-9e show coupling configuration examples. The nebulising element 1 can be placed vertically, as illustrated in Figure 9b, and associated to the top surface of the porous element 5, or it can be placed horizontally, as illustrated in Figure 9a, and associated to the side surface of the porous element 5. In this latter configuration, the nebulising element 1 forms a right angle with the axis 5a of the porous element 5. Other configurations have the nebulising element 1 so that it is angled compared to the axis 5a of the porous element 5, so as to form an acute angle (Figures 9d and 9e) or an obtuse angle (Figure 9c).

Two more alternative configurations are illustrated in the Figures 13a and 13b: in both, the second end 16b of the capillary channel 16 is inserted inside the porous element 5. To create this type of coupling, the second end 16b of the capillary channel 16 will preferably be pointed.

For these latter two configurations, it may not be necessary to use the pressure elastic bodies 19 described previously.

The dimensions of the nebulising element 1 are quite limited, with total side dimensions varying preferably between 6 and 15 mm, both in terms of length and width, and a total height that will preferably be within the range of 2-3 mm. The capillary channel 16 has a variable length, preferably between 2 and 4 mm, while the membrane 9 has lateral dimensions between 4 and 6 mm.

The capillary channel 16 needs to have a cross dimension that is less than the cross dimension of the membrane 9.

The central perforated area 11 of the membrane 9 will preferably have a diameter that can vary from between 1 and 4 mm.

Given the reduced dimensions, the best material to be used - at least for creating the first support 6 - is quartz, which allows for extremely precise operations. Alternatively, it is possible to use crystal material, amorphous material, metal or plastic. The supports can be created using the MEMS technique, mechanically, or using moulding processes. The various processing operations will affect the tolerance that can be obtained on the item.

In any case, if the material used has a low degree of wetting tension and therefore, it can slow down the capillary action movement of the liquid, the surfaces of the supports and in particular, the one coming into contact with the liquid, should be treated to obtain sufficient wetting tension.

Alternative configurations are shown in Figures 2, 7 and 10. These configurations require the nebulising element 1 to be symmetrical and able to be coupled selectively with several porous elements 5. In the configurations shown in Figures 2 and 10, the nebulising element 1 has at least two capillary channels 16, while in the configuration shown in Figure 7, the nebulising element 1 has no capillary channels 16. In the configuration illustrated in Figures 2 and 10, the channels 16 are located symmetrically compared to the collector 13. Alternative solutions may also include more than two channels 16, preferably set out on the same level and not necessary aligned with or symmetrical to the collector 13.

Each capillary channel 16 is activated individually, so as to be able to place the collector 13 in fluid communication with a single porous element 5 at a time. The capillary channel 16 is activated by physically moving the nebulising element 1 along a direction F, limited to within the support surface of the nebulising element 1 itself. The process is the same for the nebulising element 1 according to the configuration in Figure 7.

Each porous element 5 is partially immersed inside a respective reservoir 4, with each reservoir containing a liquid with different characteristics, such as different fragrances.

For nebulising elements 1 that can be coupled to more than one porous element 5, the capillary opening 14 for incoming liquid is level with the second end 16b of the enabled capillary channel 16 from time to time, while the air outlet opening 15 is level with one or more of the second ends 16b of the capillary channels 16 that have not been activated.

In this way, whatever the geometry of the nebulising element, and as well as an opening for incoming liquid and an opening to issue nebulised liquid, there is always an air outlet opening that also aids air circulation inside the collector 13.

The nebulising element 1 that is the subject of this invention can be connected to an emanator device 2 for liquid substances.

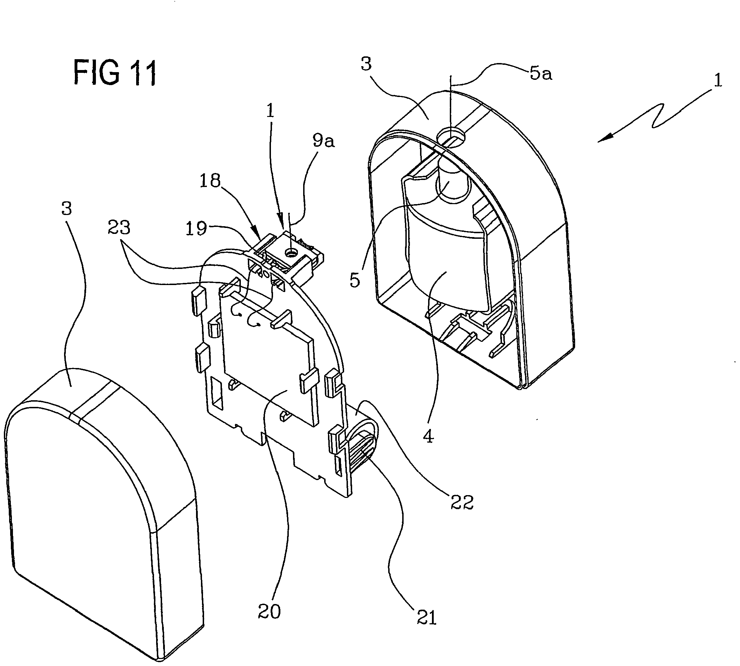

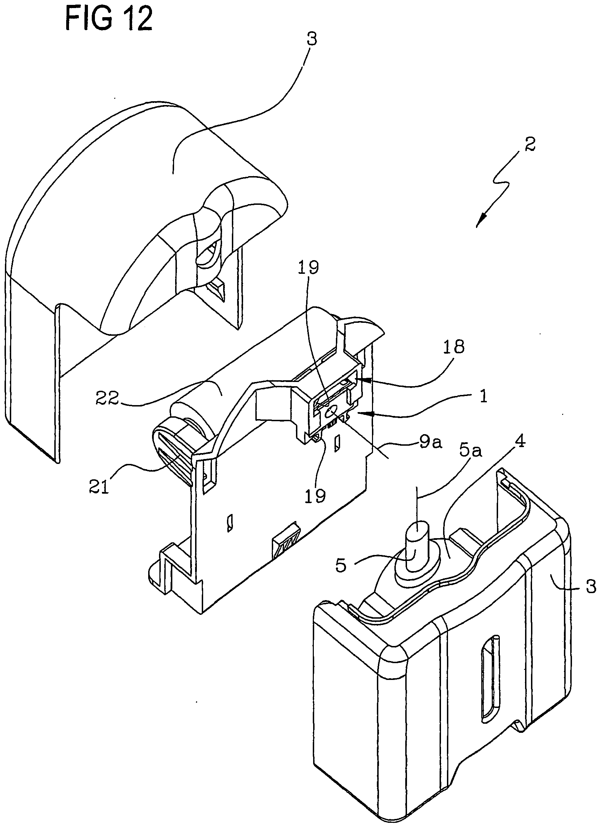

As illustrated in Figures 11 and 12, the emanator device 2 includes at least one box container 3, at least one reservoir 4 to hold the liquid to be diffused and inside which a porous element 5 is partly immersed, a source of electric power, such as a battery 22 for example, suitably secured to the plastic part with special supports 21, and the nebulising element 1 that is the subject of this invention.

The nebulising element 1 is preferably housed inside seating 18 that has been placed vertically or horizontally to the axis 5a of the porous element 5. In other words, the seating 18 is positioned in such a way as to allow the nebulising element 1 to rest on a surface that is parallel or perpendicular to the development axis 5a of the porous element 5 to which it is coupled.

Figure 11 shows an initial configuration for the emanator device 2 that is the subject of this invention, where the nebulising element 1 is set on a surface that is perpendicular to the axis 5a of the porous element 5. In this configuration, the vibration axis 9a of the membrane 9 is parallel to the axis of the porous element 5. The capillary channel 13 interferes with the side surface of the porous element 5. In this way, the oscillation of the membrane 9 is in no way constrained by the presence of the porous element 5, since the vibration plane of the membrane 9 is different from the coupling surface between the capillary channel 16 and the porous element 5, and more specifically, it is perpendicular.

Figure 12 shows a second configuration for the emanator device 2 that is the subject of this invention, where the nebulising element 1 is set out on a surface parallel to the axis 5a of the porous element 5. In this configuration, the vibration axis 9a of the membrane 9 is perpendicular to the axis of the porous element 5. The capillary channel 13 interferes with the upper surface of the porous element 5, so as not to obstruct the vibration of the membrane 9, even in this case.

In this case too, the vibration plane of the membrane 9 is different from the coupling surface between the capillary channel 16 and the porous element 5, and more specifically, it is perpendicular.

Advantageously, the seating 18 contains the aforementioned elastic bodies 19 that keep the nebulising element 1 in constant contact with the porous element 5.

The elastic bodies 19 increase the disengagement between the nebulising element 1 and the device 2, so as to insulate it better against any outside disturbance that could interfere with the oscillation of the membrane 9, sifting vibration frequency and therefore preventing efficient nebulisation.

The emanator devices 2 fitting the nebulising element 1 set on a surface that is perpendicular to the axis 5a of the porous element 5 (as per the first configuration described here above and illustrated in Figure 11) can be changed to emanator devices with multiple refills.

For multiple-refill devices, the nebulising element 1 with two or more capillary channels 16 or several capillary openings 14, is selectively moved along the direction F shown in Figure 10 to couple with the porous element 5, which is soaked in the required substance.

Whichever configuration is used for the emanator device 2, the opening 12 of the second support 7 will always face outwards.

Due to capillary action, the liquid flows from the porous element 5 to inside the collector 13, where a liquid collection basin is created. When the device 2 is enabled, the piezoelectric element vibrates and causes the membrane 9 to vibrate. During its oscillation, the membrane 9 is wetted by the liquid in the collector 13. Capillary action causes the liquid to rise up through the holes in the membrane 9 and the single drops that form are launched and nebulised to the outside, through the opening 12 in the second support 7.

One of the functions of the capillary channel 16 is as a childproof safety system. In fact, the presence of the capillary channel 16 keeps the porous element 5 away from the nebulising area and therefore, from the opening 12 of the second support 7, which has to be visible and exposed to allow the substance to be diffused. With the channel 16, the reservoir 4 and the porous element 5 can be concealed inside the device 2, in a totally closed position that cannot be accessed by children, so as to prevent any of the liquid to be diffused from being accidentally swallowed.

The nebulising element that is the subject of this invention presents sigificant advantages.

The coupling between the nebulising element and the porous element is stable and will not change the vibration frequency of the membrane.

The offset layout between the membrane and the porous element in no way invalidates the functional operation of the nebulising element; it allows the membrane to vibrate at an operating frequency that aids a nebulisation which is both effective and constant over time, without the need to perform a frequency sweep through the electronic circuit 20. In this way, the device is able to operate at a single oscillating frequency.

The position of the porous element, which is offset compared to the membrane's vibration axis, means that the characteristic operating frequency of the membrane is only defined by the configuration of the nebulising element structure and not by the characteristics for coupling the membrane and the porous element, which are not easily defined.

The liquid flows along the channel and then reaches the collector exclusively due to capillary action, thanks to the particularly limited dimensions of the nebulising element and to the presence of an air outlet opening.

In fact, this latter allows air to circulate inside the chamber, facilitating the liquid's movement by capillary action as well as its natural diffusion within the surrounding environment. The presence of an air outlet opening that is separate from the opening through which the liquid is nebulised into the environment, means it is possible to reduce the power needed to nebulise the liquid into the environment.

The nebulising element conceived in this manner is a highly flexible system since it can be coupled to the porous element at different angles, making it possible to use different forms of nebulising device inside which the nebulising element that is the subject of this invention can be inserted.

The nebulising element that is the subject of this invention is simple in structural terms, small, easy to industrialise and cheap to produce.

## Claims

1. A nebulizing element for liquid substances that can be coupled to procurement means for a liquid, such as a porous element (5), comprised of:
- at least one membrane (9) with a microperforated area (11);
- at least one piezoelectric element (10) electrically connected to an electronic circuit (20) powered electrically; the above mentioned piezoelectric element (10) is coupled with the above named membrane (9) to cause it to vibrate along an axis (9a) that is perpendicular to the position of the membrane (9) itself;
- at least one first (6) and one second (7) support that can be placed over one another to define, on the interior, a container (8) to contain the aforementioned membrane (9) and piezoelectric element (10); said support (7) presenting an opening (12) through which the nebulized liquid is emitted;
- at least one collector (13) for the liquid that, due to capillary action, flows from the porous element (5) to said collector (13);
**characterized in that** said collector (13) is positioned between said first support (6) and the membrane (9) and further comprises:
- at least one capillary opening (14), for the incoming liquid to be nebulized, at least a part of which rests upon the porous element (5);
- and at least one outlet opening (15), separate from the opening (12) through which the liquid is nebulized into the environment, to allow air circulation inside the collector (13).

2. A nebulizing element according to one of the previous claims, further comprising, placed one on top of the other in sequence, the above named first support (6), membrane (9) - including the perforated area (11) - piezoelectric element (10) and second support (7) with an opening (12) in correspondence to the perforated area (11) of the membrane (9).

3. A nebulizing element according to one of the previous claims, wherein the aforementioned collector (13) is created on the first support (6) and is defined by said first support (6) and by the aforementioned perforated membrane (9).

4. A nebulizing element according to one of the previous claims, wherein there is at least one capillary channel (16) placing the collector (13) and the porous element (5) in fluid communication with one another.

5. A nebulizing element according to claim 4), wherein the aforementioned capillary channel (16) includes a first end (16a) communicating with the liquid collector (13) and a second end (16b), opposite to the first, that can be coupled with the porous element (5).

6. A nebulizing element according to claim 5), wherein the above named capillary opening (14) for incoming liquid coincides with the second end (16b) of the aforementioned capillary channel (16).

7. A nebulizing element according to claim 4) or 5), wherein the aforementioned capillary channel (16) is defined by the above named first support (6) and by said second (7) support.

8. A nebulizing element according to claim 4) or 5), wherein the above named capillary channel (16) is defined by the first support (6) and by at least one cover plate (17).

9. A nebulizing element according to any one of the claims from 4) to 8), wherein said capillary channel (16) interferes with the porous element (5) to aid the entry of liquid into the collector (13) through capillary action.

10. A nebulizing element according to claims 5) and 9), wherein the second end (16b) of the aforementioned capillary channel (16) rests against a coupling surface of said porous element (5); the above named second end (16b) being countershaped to the coupling surface of the porous element (5).

11. A nebulizing element according to claims 5) and 9), wherein the second end (16b) of the aforementioned capillary channel (16) is pointed and penetrates the above named porous element (5).

12. A nebulizing element according to any one of the claims from 4) to 10), further comprising at least two capillary channels (16) that can be selectively coupled with a respective porous element (5); each capillary channel (16), when selectively enabled, places the connector (13) in fluid communication with the porous element (5) impregnated with the liquid to be nebulized.

13. A nebulizing element according to the claims 5) and 12), wherein when one of the two capillary channels (16) is selectively enabled, the outlet opening (15) to aid air circulation coincides with the second end (16b) of at least one deactivated capillary channel (16).

14. A nebulizing element according to any one of the previous claims, wherein the vibration axis (9a) of said membrane (9) is parallel to the axis (Sa) of said porous element (5).

15. A nebulizing element according to one of the claims from 1) to 13), wherein the vibration axis (9a) of said membrane (9) is perpendicular to the axis (Sa) of said porous element (5).

16. A nebulizing element according to any one of the previous claims, further comprising the fact that the height of the collector (13) is between 5 and 150 mµ, preferably between 80 and 120 mµ.

## Patentansprüche

1. Verneblerelement für flüssige Substanzen, das mit einem Beschaffungsmittel für eine Flüssigkeit, wie zum Beispiel einem porösen Element (5), gekoppelt werden kann, umfassend:
- mindestens eine Membran (9) mit einem mikroperforierten Gebiet (11);
- mindestens ein piezoelektrisches Element (10), das mit einer elektrisch betriebenen elektronischen Schaltung (20) elektrisch verbunden ist; wobei das obengenannte piezoelektrische Element (10) mit der obengenannten Membran (9) gekoppelt ist, um zu bewirken, dass sie entlang einer Achse (9a) schwingt, die senkrecht zur Position der Membran (9) selbst verläuft;
- mindestens einen ersten (6) und einen zweiten (7) Halter, die übereinander platziert werden können, um in dem Inneren einen Behälter (8) zur Aufnahme der obengenannten Membran (9) und des obengenannten piezoelektrischen Elements zu definieren; wobei der Halter (7) eine Öffnung (12) präsentiert, durch die die vernebelte Flüssigkeit abgegeben wird;
- mindestens eine Sammeleinrichtung (13) für die Flüssigkeit, die aufgrund von Kapillarwirkung von dem porösen Element (5) zu besagter Sammeleinrichtung (13) fließt;
**dadurch gekennzeichnet, dass** besagte Sammeleinrichtung (13) zwischen dem ersten Halter (6) und der Membran (9) positioniert ist und es ferner umfasst:
- mindestens eine Kapillaröffnung (14) für die eintretende zu vernebelnde Flüssigkeit, wobei zumindest ein Teil derselben auf dem porösen Element (5) ruht;
- und mindestens eine Auslassöffnung (15), separat von der Öffnung (12), durch die die Flüssigkeit in die Umgebung vernebelt wird, um eine Luftzirkulation innerhalb der Sammeleinrichtung (13) zu ermöglichten.

2. Verneblerelement nach einem der vorangehenden Ansprüche, ferner umfassend, platziert übereinander in Abfolge, den obengenannten ersten Halter (6), die Membran (9) - enthaltend das perforierte Gebiet (11) - das piezoelektrische Element (10) und den zweiten Halter (7) mit einer Öffnung (12) in Übereinstimmung mit dem perforierten Gebiet (11) der Membran (9).

3. Verneblerelement nach einem der vorangehenden Ansprüche, **dadurch gekennzeichnet, dass** die obengenannte Sammeleinrichtung (13) auf dem ersten Halter (6) gebildet ist und von dem ersten Halter (6) und von der obengenannten perforierten Membran (9) definiert ist.

4. Verneblerelement nach einem der vorangehenden Ansprüche, **dadurch gekennzeichnet, dass** mindestens ein Kapillarkanal (16) vorgesehen ist, der die Sammeleinrichtung (13) und das poröse Element (5) in Flüssigkeitsverbindung miteinander bringt.

5. Verneblerelement nach Anspruch 4, **dadurch gekennzeichnet, dass** der vorangehend genannte Kapillarkanal (16) ein erstes Ende (16a), das mit der Flüssigkeitssammeleinrichtung (13) in Verbindung steht, und ein zweites Ende (16b) gegenüber dem Ersten enthält, das mit dem porösen Element (5) gekoppelt werden kann.

6. Verneblerelement nach Anspruch 5, **dadurch gekennzeichnet, dass** die obengenannte Kapillaröffnung (14) für eintretende Flüssigkeit mit dem zweiten Ende (16b) des obengenannten Kapillarkanals (16) zusammenfällt.

7. Verneblerelement nach Anspruch 4 oder 5, **dadurch gekennzeichnet, dass** der obengenannte Kapillarkanal (16) von dem obengenannten ersten Halter (6) und dem zweiten Halter (7) definiert ist.

8. Verneblerelement nach Anspruch 4 oder 5, **dadurch gekennzeichnet, dass** der obengenannte Kapillarkanal (16) von dem ersten Halter (6) und von mindestens einer Abdeckplatte (17) definiert ist.

9. Verneblerelement nach einem der Ansprüche 4 bis 8, **dadurch gekennzeichnet, dass** der Kapillarkanal (16) auf das poröse Element (5) trifft, um beim Eintritt von Flüssigkeit in die Sammeleinrichtung (13) durch Kapillarwirkung zu helfen.

10. Verneblerelement nach Anspruch 5 und 9, **dadurch gekennzeichnet, dass** das zweite Ende (16b) des obengenannten Kapillarkanals (16) an einer Koppelfläche des porösen Elements (5) anliegt, wobei das obengenannte zweite Ende (16b) entgegengesetzt zur Kopplungsfläche des porösen Elements (5) gestaltet ist.

11. Verneblerelement nach Ansprüchen 5 und 9, **dadurch gekennzeichnet, dass** das zweite Ende (16b) des obengenannten Kapillarkanals (16) spitz ist und in das obengenannte poröse Element (5) eindringt.

12. Verneblerelement nach einem der Ansprüche 4 bis 10, ferner umfassend mindestens zwei Kapillarkanäle (16), die mit einem jeweiligen porösen Element (5) wahlweise gekoppelt werden können, wobei jeder Kapillarkanal (16) bei wahlweiser Aktivierung die Sammeleinrichtung (13) in Flüssigkeitsverbindung mit dem porösen Element (5) platziert, das mit der zu vernebelnden Flüssigkeit getränkt ist.

13. Verneblerelement nach den Ansprüchen 5 und 12, **dadurch gekennzeichnet, dass** bei wahlweiser Aktivierung von einem der beiden Kapillarkanäle (16) die Auslassöffnung (15) zur Unterstützung der Luftzirkulation mit dem zweiten Ende (16b) von mindestens einem deaktivierten Kapillarkanal (16) zusammenfällt.

14. Verneblerelement nach einem der vorangehenden Ansprüche, **dadurch gekennzeichnet, dass** die Schwingungsachse (9a) der Membran (9) parallel zur Achse (Sa) des porösen Elements (5) verläuft.

15. Verneblerelement nach einem der Ansprüche 1 bis 13, **dadurch gekennzeichnet, dass** die Schwingungsachse (9a) von besagter Membran (9) senkrecht zur Achse (Sa) von besagtem porösen Element (5) verläuft.

16. Verneblerelement nach einem der vorangehenden Ansprüche, ferner umfassend die Tatsache, dass die Höhe der Sammeleinrichtung (13) zwischen 5 und 150 µm, vorzugsweise zwischen 80 und 120 µm, beträgt.

## Revendications

1. Elément de nébulisation pour substances liquides pouvant être raccordé à un moyen d'approvisionnement pour un liquide, tel qu'un élément poreux (5), constitué :
- d'au moins une membrane (9) avec une zone micro-perforée (11) ;
- d'au moins un élément piézo-électrique (10) relié électriquement à un circuit électronique (20) alimenté électriquement ; ledit élément piézo-électrique (10) est raccordé à ladite membrane (9) pour l'amener à vibrer le long d'un axe (9a) qui est perpendiculaire à la position de la membrane (9) elle-même ;
- d'au moins un premier (6) et un deuxième (7) support qui peuvent être placés l'un au-dessus de l'autre pour définir, à l'intérieur, un récipient (8) pour contenir ladite membrane (9) et ledit élément piézo-électrique (10) ; ledit support (7) présentant une ouverture (12) à travers laquelle le liquide nébulisé est émis ;
- d'au moins un collecteur (13) pour le liquide qui, par effet de capillarité, s'écoule de l'élément poreux (5) jusqu'audit collecteur (13) ;
**caractérisé en ce que** ledit collecteur (13) est positionné entre ledit premier support (6) et la membrane (9) et comprend en outre :
- au moins une ouverture capillaire (14), pour le liquide entrant devant être nébulisé, dont au moins une partie repose sur l'élément poreux (5) ;
- et au moins une ouverture de sortie (15), séparée de l'ouverture (12) à travers laquelle le liquide est nébulisé dans l'environnement, pour permettre l'écoulement de l'air à l'intérieur du collecteur (13).

2. Elément de nébulisation selon l'une des revendications précédentes, comprenant en outre, placés l'un au-dessus de l'autre de manière séquencée, ledit premier support (6), la membrane (9) - y compris la zone perforée (11) - l'élément piézo-électrique (10) et le deuxième support (7) avec une ouverture (12) en correspondance avec la zone perforée (11) de la membrane (9).

3. Elément de nébulisation selon l'une des revendications précédentes, où ledit collecteur (13) est créé sur le premier support (6) et est défini par ledit premier support (6) et par ladite membrane perforée (9).

4. Elément de nébulisation selon l'une des revendications précédentes, où se trouve au moins un canal capillaire (16) plaçant le collecteur (13) et l'élément poreux (5) en communication fluide l'un avec l'autre.

5. Elément de nébulisation selon la revendication 4, où ledit canal capillaire (16) précité comporte une première extrémité (16a) communiquant avec le collecteur de liquide (13) et une deuxième extrémité (16b), opposée à la première, qui peut être raccordée à l'élément poreux (5).

6. Elément de nébulisation selon la revendication 5, dans lequel ladite ouverture capillaire (14) pour le liquide entrant coïncide avec la deuxième extrémité (16b) dudit canal capillaire (16).

7. Elément de nébulisation selon la revendication 4 ou 5, ledit canal capillaire (16) est défini par ledit premier support (6) et par ledit deuxième (7) support.

8. Elément de nébulisation selon la revendication 4 ou 5, où ledit canal capillaire (16) est défini par le premier support (6) et par au moins une plaque de couverture (17).

9. Elément de nébulisation selon l'une quelconque des revendications 4 à 8, où ledit canal capillaire (16) interfère avec l'élément poreux (5) pour faciliter l'entrée du liquide dans le collecteur (13) par effet de capillarité.

10. Elément de nébulisation selon les revendications 5 et 9, où la deuxième extrémité (16b) dudit canal capillaire (16) repose contre une surface de raccordement dudit élément poreux (5) ; ladite deuxième extrémité (16b) étant formée contre la surface de raccordement de l'élément poreux (5).

11. Elément de nébulisation selon les revendications 5 et 9, où la deuxième extrémité (16b) dudit canal capillaire (16) est pointue et pénètre dans ledit élément poreux (5).

12. Elément de nébulisation selon l'une des revendications 4 à 10, comprenant en outre au moins deux canaux capillaires (16) qui peuvent être raccordés de manière sélective à un élément poreux (5) ; chaque canal capillaire (16), lorsqu'il est sélectivement activé, place le connecteur (13) en communication fluide avec l'élément poreux (5) imprégné du liquide à nébuliser.

13. Elément de nébulisation selon les revendications 5 et 12, où lorsque l'un des deux canaux capillaires (16) est activé de manière sélective, l'ouverture de sortie (15) pour faciliter la circulation de l'air coïncide avec la deuxième extrémité (16b) d'au moins un canal capillaire désactivé (16).

14. Elément de nébulisation selon l'une quelconque des revendications précédentes, où l'axe de vibration (9a) de ladite membrane (9) est parallèle à l'axe (Sa) dudit élément poreux (5).

15. Elément de nébulisation selon l'une des revendications 1 à 13, où l'axe de vibration (9a) de ladite membrane (9) est perpendiculaire à l'axe (Sa) dudit élément poreux (5).

16. Elément de nébulisation selon l'une quelconque des revendications précédentes, comprenant en outre le fait que la hauteur du collecteur (13) se trouve entre 5 et 150 mµ, de préférence entre 80 et 120 mµ.
